# EUROPEAN PATENT SPECIFICATION

(11) **EP 1 677 840 B1**
(45) Date of publication and mention of the grant of the patent: **20.01.2010**
(21) Application number: 04810335.2
(22) Date of filing: 05.11.2004
(51) Int. Cl.: A61L 12/14, C11D 3/00, A61K 47/38

(54) **METHODS OF INHIBITING THE ADHERENCE OF LENSES TO THEIR PACKAGING MATERIALS**
METHODEN ZUR VERMEIDUNG DES KLEBENS VON LINSEN AN IHREN VERPACKUNGEN
PROCEDE POUR REDUIRE L'ADHERENCE DE LENTILLES A LEUR MATERIAU D'EMBALLAGE

(30) Priority: 05.11.2003 US 517434 P; 05.11.2004 US 982231
(43) Date of publication of application: 12.07.2006
(73) Proprietor: Johnson & Johnson Vision Care, Inc., Jacksonville, FL 32256 (US)
(72) Inventor: FORD, James D., Orange Park, Florida 32003 (US); MOLOCK, Frank, Orange Park, Florida 32003 (US); ALLI, Azaam, Jacksonville, Florida 32256 (US)
(74) Representative: Tunstall, Christopher Stephen
(86) International application number: PCT/US2004/036812
(87) International publication number: WO 2005/044322

(56) References cited:
- WO-A-00/19981
- WO-A-02/45759
- US-A- 3 954 644
- US-A1- 2003 130 144
- PATENT ABSTRACTS OF JAPAN vol. 012, no. 427 (P-784), 11 November 1988 (1988-11-11) & JP 63 159821 A (TOME SANGYO KK), 2 July 1988 (1988-07-02)
- PATENT ABSTRACTS OF JAPAN vol. 2003, no. 11, 5 November 2003 (2003-11-05) & JP 2003 183157 A (LION CORP), 3 July 2003 (2003-07-03)

## Description

This invention related to packaging solutions for use with contact lenses and methods for their use.

### RELATED APPLICATIONS

This application claims priority from a provisional patent application, U.S. Pat. App. No. 60/517,434 of the same title, filed on November 5, 2003.

### BACKGROUND

Contact lenses have been used commercially to improve vision since the 1950s. The first contact lenses were made of hard materials. Although these lenses are currently used, they are not suitable for all patients due to their poor initial comfort. Later developments in the field gave rise to soft contact lenses, based upon hydrogels, which are extremely popular today. These lenses have higher oxygen permeabilities and such are often more comfortable to wear than contact lenses made of hard materials. However, these new lenses are not without problems.

Contact lenses with high oxygen permeabilites are typically made of hydrophobic materials. The packaging for contact lenses are also made of hydrophobic materials. When one hydrophobic surface comes in contact with another, the surfaces stick to each other. The sticking of a contact lens to its packaging creates many problems. First the packaging is thicker and more rigid than the soft lenses contained therein. If a lens sticks to the packaging, when the user tries to remove the lens, the lens often tears and must be discarded. One solution to this problem is to place a hydrophilic additive such as a surfactant, into the lens packaging solution. However many surfactants that have been used to solve this problem do not prevent the sticking of contact lenses to their packaging. In addition, some surfactants do not completely dissolve in lens packaging solutions and have unfavorable interactions with the lens when they are stored over a period of time. Still further, some of the surfactants distort the physical properties of the lens, such as the diameter, the base curve, or the water content of the lens. Therefore there is a need for methods of inhibiting the adherence of contact lenses to their packaging. It is this need that is met by the following invention.

### DETAILED DESCRIPTION OF THE INVENTION

This invention includes a method of inhibiting the adherence of a silicone hydrogel or flurohydrogel lens to hydrophobic packaging materials comprising storing said silicone hydrogel or fluorohydrogel lens in a packaging solution comprising an effective amount of an appropriate surfactant, wherein the appropriate surfactant is methyl cellulose.

As used herein, the "packaging solutions" of the invention may be water-based solutions. Typical solutions include, without limitation, saline solutions, other buffered solutions, and deionized water. The preferred aqueous solution is deioinized water or saline solution containing salts including, without limitation, sodium chloride, sodium borate, sodium phosphate, sodium hydrogenphosphate, sodium dihydrogenphosphate, or the corresponding potassium salts of the same. These ingredients are generally combined to form buffered solutions that include an acid and its conjugate base, so that addition of acids and bases cause only a relatively small change in pH. The buffered solutions may additionally include 2-(N-morpholino)ethanesulfonic acid (MES), sodium hydroxide, 2,2-bis(hydroxymethyl)-2,2',2"-nitrilotriethanol, n-tris(hydroxymethyl)methyl-2-aminoethanesulfonic acid, citric acid, sodium citrate, sodium carbonate, sodium bicarbonate, acetic acid, sodium acetate, ethylenediamine tetraacetic acid and the like and combinations thereof. Preferably, the solution is a borate buffered or phosphate buffered saline solution or deionized water.

As used herein the term "storing" refers to contacting the soft lens with the packaging solution. Storing includes but is not limited to processing the lenses during manufacture or sterilizing soft lenses at a particular temperature and pressure, and subsequently maintaining those lenses at about room temperature prior to use by a consumer. The packaging solutions of the invention may be used to sterilize soft lenses in their packaging. Sterilization conditions include heating a sealed package for a period of time at a temperature and a pressure known to kill microorganisms. An example of such conditions is heating a sealed package for about 30 minutes at about 121°C and at 10-16 psi. When sterilizing the soft lenses with their packaging solutions, any adverse interactions between the appropriate surfactant and the lens are critical because the normal four year shelf life of the sealed lenses. Examples of the use of these packaging solutions in the manufacturing of soft lenses, include but are not limited to hydrating, inspecting or transferring those lenses throughout a manufacturing line. Sterilization and subsequent storage of packaged soft lenses is the preferred method of practicing this invention.

As used herein, an "appropriate surfactant" is a surfactant that inhibits the adherence to the hydrophobic packaging material but does not degrade the hydrophobic packaging material, or change the physical parameters of the lens such as lens diameter, base curve or water content. It is preferred that water content of soft lenses packaged with the appropriate surfactant neither increase or decrease greater than 1 %, more preferably greater than 0.8%.

Methyl cellulose is a polymer and the preferred molecular weight of that polymer is greater than 750,000 daltons, more preferably about 1,000,000 daltons. The preferred supplier for methyl cellulose is Fisher Scientific's brand M352 that is methoxylated 27.5% to 31.5% and a 2% solution thereof has a viscosity of 3,000 to 5,600 centipoise. Attempts to used other surfactants, including but not limited to Dextran 70, hydroxy ethyloellulose, hydroxypropyl methycellulose, hydroxymethyl cellulose, hydroxyethyl methylcellulose, and Glucamate DOE 120 were made. However none of those surfactants were suitable due to either their interaction with either the lens (for example distorting the dimensions of the lens or interacting with the lens material) or the hydrophobic packaging.

The term "effective amount" refers to the amount of appropriate surfactant (measured as weight percentage of the total weight of the packaging solution) necessary to prevent sticking of lenses to their packaging. The effective amount is about 0.001% to about 0.1%, more preferably about 0.001% to about 0.05%. The most preferred effective amount is about 0.005 % (50 ppm) to about 0.01 % (100 ppm).

The appropriate surfactant of the invention may be combined with any known active or carrier components useful for lens packaging solutions. Suitable additional ingredients include but are not limited to antibacterial agents, anti-dryness agents, such a polyvinyl alcohol, polyvinyl pyrrolidone, and dextran, tonicity agents, and combinations thereof.

As used herein "soft lens" refers to an ophthalmic device that resides in or on the eye. These devices can provide optical correction or may be cosmetic. The term lens includes but is not limited to soft contact lenses, intraocular lenses, overlay lenses, ocular inserts, and optical inserts. Soft contact lens formulations are disclosed in US Patent No. 5,710,302, WO 9421698, EP 406161, JP 2000016905, U.S. Pat. No. 5,998,498, US Pat. App. No. 09/532,943, U.S. Patent No. 6,087,415, U.S. Pat. No. 5,760,100, U.S. Pat. No.5,776,999, U.S. Pat. No. 5,789,461, U.S. Pat. No. 5,849,811, and U.S. Pat. No. 5,965,631.

The particularly preferred lenses of the inventions made from etafilcon A, genfilcon A, galifilcon A, senofilcon A, lenefilcon A, lotrfilcon A, lotrifilcon B, balifilcon A, or polymacon. More particularly preferred lenses of the invention made from genfilcon A, galifilcon A, senofilcon A, lenefilcon A, lotrfilcon A, lotrifilcon B, or balifilcon A,. The most preferred lenses include but are not limited to galifilcon A, senofilcon A, and lenses disclosed in US 2006/0007391, U.S. Patent No. 6,822,016, , U.S. Patent No. 6,087,415, U.S. Pat. No. 5,760,100, U.S. Pat. No.5,776, 999, U.S. Pat. No. 5,789,461, U.S. Pat. No. 5,849,811, and U.S. Pat. No. 5,965,631.

"Hydrophobic packaging materials," refer to substances that are used to prepare containers for manufacturing lenses prior to their use by an end user. These packaging materials are discarded by the user after the soft contact lens is placed in the eye of a user. Examples of hydrophobic packaging materials include but are not limited to polypropylene, polyethylene, nylons, olefin co-polymers, acrylics, rubbers, urethanes, polycarbonates, or fluorocarbons. The preferred materials are metallocenes polymers and co-polymers made of polypropylene, polyethylene, having a melt flow range of about 15 g/10 minutes to about 44 g/10 minutes as determined by ASTM D-1238. Containers made from hydrophobic packaging material may be in many forms. These containers may store a single lenses or many lenses. An example of a single lens storage unit is a blister package, such as the packages disclosed in the following publications, U.S. Pat. Nos. D435,966 S; 4,691,820; 5,467,868; 5,704,468; 5,823,327; 6,050,398. Examples of multiple lens storage units include the hydrophobic molds that are used to produce contact lenses as shown in U.S. Pat. No. 4,640,489. Other examples include trays, or other containers used in the process of producing soft lenses.

### EXAMPLES

The following abbreviations are used in the examples below:

| | |
|---|---|
| DMA | N,N-dimethylacrylamide |
| DPM | dipropylene glycol monomethyl ether |
| HEMA | 2-hydroxyethyl methacrylate |
| mPDMS | 800-1000 MW monomethacryloxypropyl terminated polydimethylsiloxane |
| Norbloc | 2-(2'-hydroxy-5-methacrylyloxyethylphenyl)-2H-benzotriazole |
| CGI 1850 | 1:1 (wgt) blend of 1-hydroxycyclohexyl phenyl ketone and bis(2,6-dimethoxybenzoyl)-2,4-4-trimethylpentyl phosphine oxide |
| PVP 2,500-360,000 | poly(N-vinyl pyrrolidone) having a molecular weight of approximately 2,500 to 360,000 |
| Blue HEMA | the reaction product of Reactive Blue 4 and HEMA, as described in Example 4 of U.S. Pat. no. 5,944,853 |
| IPA | isopropyl alcohol |
| D3O | 3,7-dimethyl-3-octanol |
| standard packaging solution | adding 0.10 weight % of sodium borate, 0.91 weight % Boric Acid, 0.83% Sodium Chloride, 0.01% EDTA and 98.15 weight % water into a volumetric flask and was mixed at ambient temperature until all solids were dissolved. Solution A has a pH of 7.6 (measured at 20-30°C), an osmolality of 170 (measured at ca. 25°C) and a conductivity (m/S/cm) of 0.7 (measured at 20-30°C) |
| TEGDMA | tetraethyleneglycol dimethacrylate |
| TRIS | 3-methacryloxypropyltris(trimethylsiloxy)silane |
| DOE-120 | CTFA name: Polyethylene glycol 120 methyl glucose dioleate |
| EDTA | ethylenediamine tetraacetic acid |
| DI | Deionized water |
| Macromer 2 | the reaction product of described in the examples of U.S. Pat. App. Serial No. 10/028,400 filed on December 20, 2001 and entitled Antimicrobial Contact Lenses and Methods for Their Production |
| DPMA | dipropylene glycol methyl ether acetate |
| N/A | not tested |
| Big Blue | A mixture of 900 mg blue HEMA, 44.1 g HEMA, 615 mg CGI 1850 and 150 mL ethylene glycol was stirred until homogeneous and the system was degassed as described in example 1. The mixture was transferred to a large crystallizing dish and covered with a watch glass. Polymerization of the olefinic moieties was conducted under visible light for approximately 1 hour (Phillips TL20 W/03T bulbs). Upon quenching of the polymerization using oxygen, the mixture was poured into 500mL of borate- buffered saline solution and stirred for several hours until the material was transformed into a more rigid form. The liquids were decanted, and the product was washed with another 500mL of borate-buffered saline solution, the polymer was cut into several smaller pieces, and stirred in 500mL of deionized water for more than 1 hour to the point that the product became gel-like and sparingly soluble in the solvent. The mixture was then diluted with a small quantity of borate-buffered saline solution to enable better precipitation of the polymer. The mixture was filtered and washed in deionized water until the material did not appear soluble. The suspension was filtered, dried in a rotary evaporator, cut into smaller pieces and further dried until it appeared crystalline and anhydrous. The dark blue polymer was then milled into fine particles and subjected to more deionized water washings accompanied by 1 to 2 hours of stirring with each wash. Washing continued until little or no blue color was visible in solution and the product was filtered, dried at reduced pressure, and ground in a blender. |
| | GPC data for each of the polymers were obtained using both R.I and light scattering detectors. Chromatography was performed using a mixed bed GPC column (phenogel 300 mm x 7.8 mm x 5 micron (2) column (Phenomenex) having a separation range of 100 K to 10,000 K, and 0.5wt% lithium bromide in dimethylformamide as the eluent. |
| | Mn = 1.133X10⁶; Mw = 1.222X10⁶; Mz = 1.354X10⁶; polydispersity (Mw/Mn) = 1.078. |

### Lens Preparation

### Lenses G, H, J, and K

Monomer mix is prepared by blending 17.98 weight percent of GTP(Macromer 2), 28%mPDMS,14% TRIS, 26% DMA, 5% HEMA, 5% PVP (360,000 molecular weight), 2% Norbloc, 1 % TEGDMA, 0.02% Blue HEMA, 1% CGI 1850, in a blend with 80 parts of this combination with 20 parts D3O diluent. Thermoplastic contact lens molds were coated with pHEMA(great white) coating on the surface as per the method disclosed in U.S. Pat. Application No. 09/921,192 entitled "Method for Correcting Articles by Mold Transfer." Subsequently, the contact lenses were made by placing this monomer mix into thermoplastic contact lens molds, and irradiating using Philips TL20W/03T fluorescent bulbs at 70°C for about 15minutes. The molds were opened and lenses were extracted into DPM. Subsequently, the lenses were equilibrated in either DI, or a combination of 0.01 weight per of methyl cellulose in DI and 0.005 weight percent of methyl cellulose in standard packaging solution. Lenses G and H were equilibrated in DI. Lenses J and K were equilibrated in a combination of 0.01 weight per of methyl cellulose in DI and 0.005 weight percent of methyl cellulose in standard packaging solution.

### Standard Packaging Solution With and Without an a Surfactant Example 1

Lenses H, J, and K were packaged in blister packs using only packaging solution or 0.005 weight percent of methyl cellulose as listed in Table 1. Lenses G were packaged in glass vials without any surfactant. The packaged lenses were autoclaved (121 °C, 30 minutes) and visually evaluated to determine whether the lenses stuck to the packaging. The data is presented in Table 1 and indicates that methyl cellulose prevents the sticking of lenses to their packaging.

**Table 1**

| Lens Type | weight percent of methyl cellulose | percentage of lenses that are stuck to the packaging |
|---|---|---|
| G | None | 0% |
| H | 0.005 | 0% |
| J | None | 98% |
| K | 0.005 | 0% |

## Claims

1. A method of inhibiting the adherence of a silicone hydrogel or fluorohydrogel lens to hydrophobic packaging materials comprising, storing said silicone hydrogel or fluorohydrogel lens in a packaging solution comprising an effective amount of an appropriate surfactant, wherein the appropriate surfactant is methyl cellulose.

2. The method of claim 1 wherein the effective amount of the appropriate surfactant is about 0. 001 % to about 0.05%.

3. The method of claim 1 wherein the effective amount is about 0. 001 % to about 0.01 %.

4. The method of claim 1 wherein the effective amount is about 0.005%.

5. The method of claim 1 or claim 4 wherein the packaging solution comprises deionized water.

6. The method of claim 4 wherein the packaging solution comprises borate buffered or phosphate buffered saline solution.

7. The method of claim 1 wherein the appropriate surfactant does not distort the physical properties of the soft lens.

8. The method of claim 7 wherein the appropriate surfactant does not distort the diameter, base curve or water content of the soft lens.

9. The method of claim 7 wherein the appropriate surfactant does not distort the diameter or base curve of the soft lens.

10. The method of claim 7 wherein the appropriate surfactant does not distort the water content of the soft lens.

11. The method of claim 7 wherein the appropriate surfactant does not increase or decrease the water content of the lens more than about 1 %.

12. The method of claim 7 wherein the appropriate surfactant does not increase or decrease the water content of the lens more than about 0.8%.

13. The method of claim 7 wherein the appropriate surfactant does not distort the diameter of the soft lens.

14. The method of claim 1, claim 7 or claim 11 wherein the soft lenses comprise genfilcon A, galifilcon A, senofilcon A, lenefilcon A, lotrfilcon A, lotrifilcon B, or balifilcon A.

15. The method of claim 1 or claim 7 wherein the soft lenses comprise galifilcon A or senofilcon A.

## Patentansprüche

1. Verfahren zur Hemmung des Anhaftens einer Silikonhydrogel- oder Fluorhydrogel-Linse an hydrophoben Verpackungsmaterialien, welches umfasst, dass die Silikonhydrogel- oder Fluorhydrogel-Linse in einer Verpackungslösung aufbewahrt wird, die eine wirksame Menge eines geeigneten Tensids umfasst, wobei das geeignete Tensid Methylcellulose ist.

2. Verfahren nach Anspruch 1, wobei die wirksame Menge des geeigneten Tensids etwa 0,001% bis etwa 0,05% beträgt.

3. Verfahren nach Anspruch 1, wobei die wirksame Menge etwa 0,001% bis etwa 0,01% beträgt.

4. Verfahren nach Anspruch 1, wobei die wirksame Menge etwa 0,005% beträgt.

5. Verfahren nach Anspruch 1 oder Anspruch 4, wobei die Verpackungslösung entionisiertes Wasser umfasst.

6. Verfahren nach Anspruch 4, wobei die Verpackungslösung boratgepufferte oder phosphatgepufferte Kochsalzlösung umfasst.

7. Verfahren nach Anspruch 1, wobei das geeignete Tensid die physikalischen Eigenschaften der weichen Linse nicht verzerrt.

8. Verfahren nach Anspruch 7, wobei das geeignete Tensid den Durchmesser, die Basiskurve oder den Wassergehalt der weichen Linse nicht verzerrt.

9. Verfahren nach Anspruch 1, wobei das geeignete Tensid den Durchmesser oder die Basiskurve der weichen Linse nicht verzerrt.

10. Verfahren nach Anspruch 7, wobei das geeignete Tensid den Wassergehalt der weichen Linse nicht verzerrt.

11. Verfahren nach Anspruch 7, wobei das geeignete Tensid den Wassergehalt der Linse nicht mehr als etwa 1 % erhöht oder senkt.

12. Verfahren nach Anspruch 7, wobei das geeignete Tensid den Wassergehalt der Linse nicht mehr als etwa 0,8% erhöht oder senkt.

13. Verfahren nach Anspruch 7, wobei das geeignete Tensid den Durchmesser der weichen Linse nicht verzerrt.

14. Verfahren nach Anspruch 1, Anspruch 7 oder Anspruch 11, wobei die weichen Linsen Genfilcon A, Galifilcon A, Senofilcon A, Lenefilcon A, Lotrfilcon A, Lotrifilcon B oder Balifilcon A umfassen.

15. Verfahren nach Anspruch 1 oder Anspruch 7, wobei die weichen Linsen Galifilcon A oder Senofilcon A umfassen.

## Revendications

1. Procédé d'inhibition de l'adhérence d'une lentille d'hydrogel ou de fluoro-hydrogel de silicone à des matériaux de conditionnement hydrophobes comprenant les étapes consistant à stocker ladite lentille d'hydrogel ou de fluoro-hydrogel de silicone dans une solution de conditionnement comprenant une quantité efficace d'un tensioactif adapté, où le tensioactif adapté est la méthylcellulose.

2. Procédé selon la revendication 1, dans lequel la quantité efficace du tensioactif adapté se situe dans la plage allant d'environ 0,001 % à 0,05 %.

3. Procédé selon la revendication 1, dans lequel la quantité efficace se situe dans la plage allant de 0,001 % à 0,01 %.

4. Procédé selon la revendication 1, dans lequel la quantité efficace est égale à environ 0,005 %.

5. Procédé selon la revendication 1 ou la revendication 4, dans lequel la solution de conditionnement comprend de l'eau déminéralisée.

6. Procédé selon la revendication 4, dans lequel la solution de conditionnement comprend une solution saline tamponnée au borate ou tamponnée au phosphate.

7. Procédé selon la revendication 1, dans lequel le tensioactif adapté ne déforme pas les propriétés physiques de la lentille souple.

8. Procédé selon la revendication 7, dans lequel le tensioactif adapté ne déforme pas le diamètre, la courbe de base ou la teneur en eau de la lentille souple.

9. Procédé selon la revendication 7, dans lequel le tensioactif adapté ne déforme pas le diamètre ou la courbe de base de la lentille souple.

10. Procédé selon la revendication 7, dans lequel le tensioactif adapté ne déforme pas la teneur en eau de la lentille souple.

11. Procédé selon la revendication 7, dans lequel le tensioactif adapté n'augmente ni ne réduit la teneur en eau de la lentille de plus d'environ 1 %.

12. Procédé selon la revendication 7, dans lequel le tensioactif adapté n'augmente ni ne réduit la teneur en eau de la lentille de plus d'environ 0,8 %.

13. Procédé selon la revendication 7, dans lequel le tensioactif adapté ne déforme pas le diamètre de la lentille souple.

14. Procédé selon la revendication 1, 7 ou 11 dans lequel les lentilles souples comprennent du genfilcon A, du galifilcon A, du senofilcon A, du lenefilcon A, du lotrifilcon A, du lotrifilcon B, du ou balifilcon A.

15. Procédé selon la revendication 1 ou la revendication 7, dans lequel les lentilles souples comprennent du galifilcon A ou du senofilcon A.
